(19) Europäisches Patentamt
European Patent Office
Office européen des brevets

(11) Veröffentlichungsnummer: **0 043 997**
**B1**

(12) # EUROPÄISCHE PATENTSCHRIFT

(45) Veröffentlichungstag der Patentschrift: **25.09.85**

(51) Int. Cl.⁴: **C 08 K 5/42**, C 08 L 69/00, C 07 D 209/48

(21) Anmeldenummer: **81105144.0**

(22) Anmeldetag: **02.07.81**

(54) **Flammverzögerungsmittel für Polycarbonate.**

(30) Priorität: **14.07.80 US 167971**

(43) Veröffentlichungstag der Anmeldung: **20.01.82 Patentblatt 82/3**

(45) Bekanntmachung des Hinweises auf die Patenterteilung: **25.09.85 Patentblatt 85/39**

(84) Benannte Vertragsstaaten: **DE FR GB IT NL**

(56) Entgegenhaltungen: **DE - A - 2 461 144**

**Die Akte enthält technische Angaben, die nach dem Eingang der Anmeldung eingereicht wurden und die nicht in dieser Patentschrift enthalten sind.**

(73) Patentinhaber: **Mobay Chemical Corporation, Penn Lincoln Parkway West, Pittsburgh, Pennsylvania 15205 (US)**

(72) Erfinder: **Krishnan, Sivaram, Achsenstrasse 12, D-4130 Moers (DE)**
Erfinder: **Carter, Russel P., Jr., 12 Orchard Drive, New Martinsville W.VA. 26155 (US)**
Erfinder: **Kroshefsky, Robert D., 406 Carmel Drive, Aliquippa, PA 15001 (US)**

(74) Vertreter: **Gremm, Joachim, Dr. et al, Bayer AG c/o Zentralbereich Patente, Marken und Lizenzen, D-5090 Leverkusen 1, Bayerwerk (DE)**

**EP 0 043 997 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Polycarbonate, die aus Reaktionen erhalten werden, an denen organische Dihydroxy-Verbindungen und Kohlensäure-Derivate beteiligt sind, haben auf Grund ihrer ausgezeichneten mechanischen und physikalischen Eigenschaften weitgehende technische Anwendungen gefunden. Diese thermoplastischen Polymerisate eignen sich besonders gut für die Herstellung von Formteilen, bei denen es auf Schlagzähigkeit, Steifigkeit, Zähigkeit, thermische Stabilität und Masshaltigkeit sowie ausgezeichnete elektrische Eigenschaften ankommt.

Indessen zeigen diese Polymeren eine kurze, aber dennoch deutliche Brennzeit, wenn sie mit einer offenen Flamme in Berührung gebracht werden, und erfüllen in Anwendungsfällen, in denen sie möglicherweise hohen Temperaturen und/ oder Feuer ausgesetzt sein können, bestimmte spezifizierte Voraussetzungen der Schwerentflammbarkeit nicht. Hinzu kommt, dass den Polycarbonaten normalerweise zugesetzte Stabilisatoren oder andere funktionelle Additive die Brenneigenschaften des Kunststoffs weiter verändern und seinen Einsatz dort verbieten, wo Flammverzögerungsvermögen speziell gefordert wird.

Zur Erhöhung der flammwidrigen Eigenschaften wurden in Polymere solche Stabilisatoren und funktionelle Additive wie monomere Phosphate, Phosphorsäureester und Thiophosphorsäureester mit halogenierten Alkylradikalen eingearbeitet. Auch Metallsalze wurden eingesetzt, um Polycarbonate mit flammhemmenden Eigenschaften auszustatten. Weiterhin wurden bei der Herstellung aromatischer Polycarbonat-Harze zum Teil phenolische Diole eingesetzt, die am aromatischen Ring chlor- oder bromsubstituiert waren, um dadurch dem Polycarbonat-Endprodukt flammwidrige Eigenschaften zu verleihen. Beispielhaft für die Sulfonsäure-Salze und für Metallsalze sind diejenigen, die in den US-PSen 3 775 367, 4 067 846, 4 073 768, 4 075 164, 4 066 618, 4 069 201, 4 093 589, 3 933 734, 3 940 366 und 3 919 167 beschrieben wurden.

Einige dieser Flammenverzögerungsmittel werden jedoch, um Wirkung zu erzielen, in relativ grossen Mengen zugesetzt und wirken sich dadurch nachteilig auf einige der erwünschten Eigenschaften des Harz-Grudstoffs aus. Beispielsweise werden durch Zusatz grosser Mengen eines Salzes sowohl die Schlagzähigkeit als die hydrolytische Beständigkeit beeinträchtigt. Weiter unterliegen manche dieser flammhemmenden Zusätze der Gefahr der Verflüchtigung bei den hohen Verformungstemperaturen der Polycarbonate, so dass ein Zusatz eines Überschusses erforderlich wird, der wiederum eine Trübung und einen Verlust an Durchsichtigkeit zur Folge hat. Da bei den Anwendern einheitliche Bedingungen des Verformens nicht gegeben sind, ist es schwierig, ja praktisch unmöglich, die Menge des in das Polycarbonat einzuarbeitenden Zusatzes eindeutig festzulegen.

Flammenverzögerungsmittel, die eine Phthalimid-Gruppe enthalten, wurden beispielsweise in der GB-PS 2 287 934 und den US-PSen 3 873 567, 3 923 734, 3 915 930, 3 868 388, 4 087 441, 4 001 179 und 4 003 362 offenbart.

Gemäss deutscher Offenlegungsschrift Nr. 2 461 144, Seite 10, Tabelle I wird auch Lithium-N-phenylphthalimid-4'-sulfonat in Mengen von 1 Gew.% als Flammschutzmittel zugesetzt, das jedoch gemäss dieser Literaturstelle nach Brandtest UL 94 nur eine SE-II-Klassifikation aufweist.

Gegenstand der vorliegenden Erfindung sind Verbindungen der allgemeinen Formel

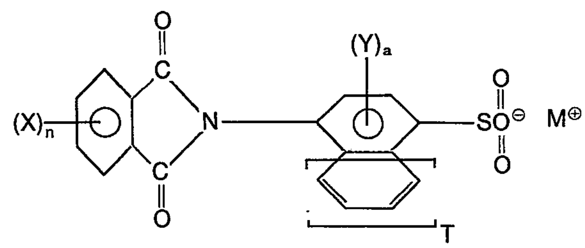

worin

X für ein Halogen,

n für 4,

Y für ein niederes Alkyl mit 1 bis 4 Kohlenstoff-Atomen oder ein Halogen-Atom,

a für eine ganze Zahl von 0 bis 4, vorzugsweise 0,

M für Natrium oder Kalium, vorzugsweise Natrium, und

T für entweder 0 oder 1 stehen.

Beispiele für Halogen sind Fluor, Brom, Chlor oder Jod, vorzugsweise Chlor oder Brom.

Gegenstand der vorliegenden Erfindung sind ausserdem thermoplastische, aromatische Polycarbonate, die 0.01 bis 1 Gew.%, vorzugsweise 0.1 bis 0.75 Gew.% an diesen Verbindungen der allgemeinen Formel enthalten.

In der vorliegenden Beschreibung ist mit «Polycarbonat-Harz» das reine Harz ohne Zusatzstoffe gemeint; demgegenüber bezeichnet «Polycarbonat» sowohl das formulierte Polycarbonat-Harz mit den darin enthaltenen Additiven als auch das fertige geformte Kunststoff-Erzeugnis.

Die bei der Anwendung der vorliegenden Erfindung zum Einsatz gelangenden Polycarbonat-Harze werden durch Reaktion phenolischer Dihydroxy-Verbindungen wie Di-(monohydroxyaryl)-alkane oder Dihydroxy-benzole und substituierter Dihydroxy-benzole mit Kohlensäure-Derivaten wie Kohlensäure-diestern, Phosgen, Bis-chlorkohlensäure-estern von Di-(monohydroxyaryl)-alkanen und Bis-chlorkohlensäureestern von Dihydroxy-benzolen und substituierten Dihydroxybenzolen hergestellt.

Die beiden Aryl-Gruppen der gemäss der Erfindung verwendeten Di-(monohydroxaryl)-alkane können gleich oder voneinander verschieden sein. Die Aryl-Gruppen können auch solche Substituenten tragen, die bei der Umwandlung in Polycarbonate nicht zur Reaktion befähigt sind, wie Halogen-Atome oder Alkyl-Gruppen, z.B. die

Gruppen Methyl-, Ethyl-, Propyl- oder tert-Butyl-. Der Alkyl-Teil der Di-(monohydroxyaryl)-alkane, der die beiden Benzol-Ringe miteinander verknüpft, kann aus einer offenen Kette oder einem cycloaliphatischen Ring bestehen und, falls erwünscht, beispielsweise durch eine Aryl-Gruppe substituiert sein.

Geeignete Di-(monohydroxyaryl)-alkane sind beispielsweise 4,4′-Dihydroxy-diphenyl-methan, 2,2-(4,4′-Dihydroxy-diphenyl)-propan, 1,1-(4,4′-Dihydroxy-diphenyl)-cyclohexan, 1,1-(4,4′-Dihydroxy-3,3′-dimethyl-diphenyl)-cyclohexan, 1,1-(2,2′-Dihydroxy-4,4′-dimethyl-diphenyl)-butan, 2,2-(2,2′-Dihydroxy-4,4′-di-tert-butyl-diphenyl)-propan oder 1,1-(4,4′-Dihydroxy-diphenyl)-1-phenyl-ethan, weiterhin auch Methan-Derivate, die neben den beiden Hydroxyaryl-Gruppen eine Alkyl-Gruppe mit mindestens zwei Kohlenstoff-Atomen und eine zweite Alkyl-Gruppe mit einem oder mehreren Kohlenstoff-Atomen tragen, wie z.B. 2,2-(4,4′-Dihydroxy-diphenyl)-butan, 2,2-(4,4′-Dihydroxy-diphenyl)-pentan (Schmelzpunkt 149–150 °C), 3,3-(4,4′-Dihydroxy-diphenyl)-pentan, 2,2-(4,4′-Dihydroxy-diphenyl)-hexan, 3,3-(4,4′-Dihydroxy-diphenyl)-hexan, 2,2-(4,4′-Dihydroxydiphenyl)-4-methyl-pentan, 2,2-(4,4′-Dihydroxy-diphenyl)-heptan, 4,4-(4,4′-Dihydroxy-diphenyl)-heptan (Schmelzpunkt 148–149 °C) oder 2,2-(4,4′-Dihydroxy-diphenyl)-tridecan. Geeignete Di-(monohydroxy-aryl)-alkane mit zwei voneinander verschiedenen Aryl-Resten sind beispielsweise 2,2-(4,4′-Dihydroxy-3-methyl-diphenyl)-propan und 2,2-(4,4′-Dihydroxy-3-methyl-3′-isopropyl-diphenyl)-butan. Geeignete Di-(monohydroxy-aryl)-alkane, deren Aryl-Reste Halogen-Atome tragen, sind beispielsweise 2,2-(3,3′,5,5′-Tetrachloro-4,4′-dihydroxy-diphenyl)-propan, 2,2-(3,3′,5,5′-Tetrabromo-4,4′-dihydroxy-diphenyl)-propan, (3,3′-Dichloro-4,4′-dihydroxy-diphenyl)-methan und 2,2′-Dihydroxy-5,5′-difluoro-diphenyl-methan. Geeignete Di-(monohydroxy-aryl)-alkane, deren die beiden Benzol-Ringe verknüpfende Alkyl-Gruppe durch einen Aryl-Rest substituiert ist, sind beispielsweise (4,4′-Dihydroxy-diphenyl)-phenylmethan und 1,1-(4,4′-Dihydroxy-diphenyl)-1-phenyl-ethan.

Geeignete Dihydroxybenzole und substituierte Dihydroxybenzole sind Hydrochinon, Resorcin, Brenzcatechin, Methyl-hydrochinon und dergleichen. Andere geeignete dihydroxy-aromatische Verbindungen sind 4,4′-Dihydroxy-diphenyl, 2,2′-Dihydroxy-diphenyl, Dihydroxynaphthalin, Dihydroxyanthracen und Verbindungen nachstehender Strukturformel

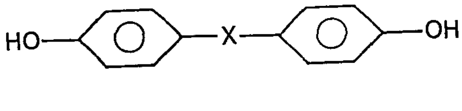

worin X für S, C, S oder S steht.

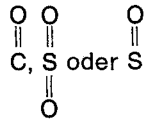

Zur Erzielung spezieller Eigenschaften können auch Gemische verschiedener Di-(monohydroxyaryl)-alkane eingesetzt werden, wobei dann gemischte Polycarbonat-Harze erhalten werden. Die bei weitem wertvollsten Polycarbonat-Harze sind diejenigen auf Bis(4-hydroxyaryl)-alkan-Basis, insbesondere auf der Basis von Bisphenol A [2,2-(4,4′-Dihydroxy-diphenyl)-propan].

Zusätzlich können kleinere Mengen tri- oder höher funktioneller aromatischer Verbindungen zur Verzweigung der Polycarbonat-Harze eingesetzt werden. Beispiele verwendbarer Verbindungen mit drei oder mehr phenolischen Hydroxyl-Gruppen sind Phloroglucin, 2,4-Dimethyl-2,4,6-tris(4-hydroxyphenyl)-heptan, 2,4,6-Trimethyl-2,4,6-tris(4-hydroxyphenyl)-heptan, 1,4,5-Tris(4-hydroxyphenyl)-benzol, 1,1,1-Tris(4-hydroxyphenyl)-ethan, Tris(4-hydroxyphenyl)-phenyl-methan, 2,2-Bis[4,4′-bis(4-hydroxyphenyl)-cyclohexyl]-propan, 2,4-Bis(4-hydroxyphenyl-isopropyl)-phenol, 2,6-Bis(2-hydroxy-5-methylbenzyl)-4-methyl-phenol, 2-(4-Hydroxyphenyl)-2-(2,4-dihydroxyphenyl)-propan, Tetrakis(4-hydroxyphenyl)-methan, Tetrakis[4-(4-hydroxyphenyl-isopropyl)-phenoxy]-methan und 1,4-Bis[(4′,4″-dihydroxytriphenyl)-methyl]-benzol. Zu den anderen funktionellen Verbindungen zählen 2,4-Dihydroxybenzoesäure, Trimellitsäure, Cyanurchlorid und 3,3-Bis(4-hydroxy-phenyl)-2-oxo-2,3-dihydroindol.

Die Polycarbonat-Harze haben ein Gewichtsmittel-Molekulargewicht von 10 000 bis 200 000 und vorzugsweise eine Schmelzflussrate (nach ASTM 1238) von 1 bis 24 g/10 min und werden mit Hilfe von nach dem Stand der Technik bekannten Verfahren, insbesondere nach den in den US-PSen 3 028 365, 2 999 846, 3 248 414, 3 153 008, 2 991 273 und 2 999 835 offenbarten Verfahren, hergestellt.

Das halogenierte aromatische Imid-Sulfonat kann durch Reaktion einer halogensubstituierten aromatischen Ortho-dicarbonsäure bzw. ihre Anhydrids mit einem substituierten oder unsubstituierten Natrium- beziehungsweise Kaliumsalz der Sulfanilsäure in äquimolaren Mengen dargestellt werden. Die Reaktion wird bei höherer Temperatur in einem geeigneten Lösungsmittel durchgeführt und liefert ein halogeniertes aromatisches Imid-Sulfonat-Salz, das für die Zwecke der Erfindung verwendbar ist.

Zusätzlich zu dem halogenierten aromatischen Imid-Sulfonat kann auch eine geringe Menge eines Polytetrafluorethylen-Polymeren als die Tropfenbildung unterdrückendes Mittel in das Polycarbonat eingearbeitet werden. Sowohl faserbildende als auch nicht-faserbildende fluorierte Polyolefin-Polymere können verwendet werden, die typischerweise durch Polymerisation fluorierter Olefine wie fluoriertes Ethylen oder fluoriertes Propylen hergestellt werden; bevorzugte Verwendung findet Polytetrafluorethylen. Die Gesamtkonzentration des fluorierten Polyolefin-Polymeren in dem Polycarbonat beträgt etwa 0,01 bis 3 Gewichts-%, vorzugsweise 0,01 bis 2 Gewichts-%, bezogen auf das Polycarbonat-Harz.

Zusätzlich zu den vorerwähnten Bestandteilen können auch andere nach dem Stand der Technik bekannte Materialien verwendet werden, um den Produkten besondere Eigenschaften zu verleihen. Beispielsweise können Pigmente oder Farbstoffe zur Bildung undurchsichtiger oder gefärbter Formteile zugesetzt werden. Zur Bildung undurchsichtiger Produkte wird überwiegend Titandioxid verwendet, dessen Zusatz die Herstellung weisser Polycarbonat-Formteile bewirkt. Falls ein gefärbtes Produkt erwünscht ist, können Pigmente wie Chromgelb und -orange sowie Chromgrün zugesetzt werden, wodurch sich verschiedenfarbige Gegenstände herstellen lassen. Ebenso können auch öllösliche Farbstoffe in das Polycarbonat zum Zweck der Einfärbung des fertigen Formteils eingearbeitet werden. Zur Einfärbung des Polycarbonats können Mindestmengen des Farbstoffs, d.h. 0,022 bis 22 g Farbstoff/kg Polycarbonat (0,01 to 10 grams per pound) erforderlich sein.

Vorzugsweise zeigt das gemäss der Erfindung hergestellte Polycarbonat einen als UL- (Underwriters' Laboratories, Inc.) 94 V-O bekannten Wert. Der UL 94-Test wird wie folgt durchgeführt:

Polycarbonat-Proben werden zu Stäben der Abmessungen 127 x 12,7 x 1,6 (oder 3,2) mm (5.00 x 0.5 x 1/16 (or 1/8) inches) geformt. Die Stäbe werden vertikal so montiert, dass die Unterseite des Probekörpers sich 305 mm über einem Streifen Watte befindet. Jeder Probestab wird einzeln mittels zweier aufeinanderfolgender Zündvorgänge von 10 s Dauer entzündet, die Brenneigenschaften nach jedem Zündvorgang werden beobachtet und danach die Probe bewertet. Zum Entzünden der Probe wird ein Bunsenbrenner mit einer 10 mm (3/8 inch) hohen blauen Flamme von Erdgas mit einem Wärmeinhalt von $3{,}73 \times 10^4$ kJ/$m^3$ (1,000 BTU per cubic foot) benutzt.

Die UL 94 V-O Klassifizierung umfasst die nachstehend beschriebenen Eigenschaften von Materialien, die gemäss der UL 94 -Vorschrift geprüft wurden. Die Polycarbonate in dieser Klasse enthalten keine Proben, die länger als 10 s nach jeder Einwirkung der Testflamme brennen; sie zeigen keine Gesamt-Flammzeit von mehr als 50 s bei der zweimaligen Flammeneinwirkung auf jeden Probensatz; sie enthalten keine Proben, die vollständig bis hinauf zu der am oberen Ende der Probe befestigten Halteklammer abbrennen; sie weisen keine Proben auf, die die unterhalb der Probe angeordnete Watte durch brennende Tropfen oder Teilchen entzünden; sie enthalten auch keine Proben, die länger als 30 s nach Entfernen der Testflamme glimmen.

Andere UL 94-Klassifizierungen bezeichnen Proben, die weniger flammwidrig und selbstverlöschend sind und die flammende Tropfen oder Teilchen abgeben. Diese Klassifizierungen werden mit UL 94 V-1 und V-2 bezeichnet. Die Polycarbonate innerhalb des Bereichs dieser Erfindung zeigen in charakteristischer Weise die für eine UL 94 V-O-Klassifizierung geforderten Eigenschaften.

Die Erfindung wird durch die folgenden Beispiele weiter erläutert, die gewisse bevorzugte Ausführungsformen beschreiben.

Beispiel 1

17,3 g Sulfanilsäure und 5,3 g Natriumcarbonat wurden in einem 500 ml-Einhals-Rundkolben gefüllt, der mit einem Magnetrührer, Stickstoff-Einlass und einem Rückflusskühler ausgerüstet war. 300 ml Dimethylformamid wurden dann in den Kolben gegeben und die Sulfanilsäure und das Natriumcarbonat in Dimethylformamid bis zum Rückfluss erhitzt; zu diesem Zeitpunkt hatte sich der grösste Teil des festen Bodenkörpers aufgelöst. Die Mischung wurde auf Raumtemperatur abkühlen lassen und über Nacht gerührt. Das Natriumsalz der Sulfanilsäure fiel aus der Lösung bei Raumtemperatur als weisser flockiger Niederschlag aus. 28,6 g Tetrachlor-phthalsäureanhydrid wurden dann in den Kolben mit dem vorher hergestellten Natriumsalz der Sulfanilsäure gefüllt. Das Gemisch wurde erhitzt, wobei eine homogene Lösung entstand. Die Lösung wurde dann 20 Minuten auf 120 °C erhitzt, und das Tetrachloro-phthalimido-benzolsulfonsäure-Natriumsalz (TCPS) begann auszufallen. Die Mischung wurde danach auf Raumtemperatur abgekühlt und filtriert. Der erhaltene Niederschlag wurde mit 250 ml Dimethylformamid und ein erhaltener Feststoff anschliessend mit 100 ml Ether gewaschen. Das verbleibende Produkt wurde zunächst über Nacht an der Luft und anschliessend im Vakuum getrocknet. Die Substanz besass einen Schmelzpunkt oberhalb von 310 °C.

Die dargestellte Verbindung hat nachstehende Strukturformel

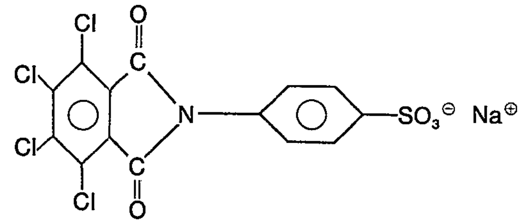

und lieferte die folgenden Werte der Elementaranalyse im Vergleich zu den theoretischen Daten (Gewichts-%):

| | C | H | N | S | Cl | Na |
|---|---|---|---|---|---|---|
| berechnet: | 36,30 | 0,87 | 3,02 | 6,90 | 30,66 | 4,97 |
| gefunden: | 36,06 | 1,14 | 3,10 | 6,90 | 30,27 | 4,72 |

Beispiel 2

Ein Polycarbonat auf der Basis von Bisphenol A mit einer Schmelzflussrate von 7,4 g/10 min wurde in Granulatform in einem Trommelmischer mit einer Menge Natrium-tetrachloro-phthalimidobenzolsulfonat (TCPS) gemischt, die einer Konzentration des Imid-Salzes von 0,5 Gewichts-%, bezogen auf das Gesamtgewicht des Polycarbonat-Harzes, entsprach.

Das Gemisch aus Harz und Salz wurde zunächst zu einer Strangpressmischung verarbeitet und dann zu 3,2 mm- (1/8″) oder 1,6 mm- (1/16″) -Proben geformt, wie sie für den UL 94 Test vorgesehen sind. Die 3,2 mm-Proben erzielten eine V-O-Bewertung mit einer durchschnittlichen Brennzeit nach dem Entfernen der Flamme von 2,4 s. Die 1,6 mm-Proben erhielten ebenfalls eine V-O-Bewertung mit einer durchschnittlichen Brennzeit nach dem Entfernen der Flamme von 3,0 s (siehe Tabelle 1).

Beispiel 3

Beispiel 2 wurde mit der Abänderung wiederholt, dass 1 Gewichts-% Natrium-tetrachloro-phthalimido-benzolsulfonat (TCPS) statt 0,5% verwendet wurde. Die Prüfung nach UL 94 ergab eine V-O-Bewertung für die 3,2 mm- (1/8″) Probe mit einer durchschnittlichen Brennzeit nach dem Entfernen der Flamme von 0,7 s. Die 1,6 mm-(1/16″) Probe erhielt eine V-O-Bewertung mit einer durchschnittlichen Brennzeit nach dem Entfernen der Flamme von 2,3 s (siehe Tabelle 1).

Tabelle I

TCPS als flammenverzögernder Zusatzstoff [a]

| TCPS-Konzentration % | UL 94-Bewertung 3,2 mm-Probe (1/8″) | 1,6 mm-Probe 1/16″) |
|---|---|---|
| 0,5 | V-O (2,4)[b] | V-O (3,0) |
| 1,0 | V-O (0,7) | V-O (2,3) |

[a] Der Zusatzstoff ist in ein Polycarbonat auf BPA-Basis eingearbeitet, das auch Kryolith und PTFE enthielt.

[b] Die Zahlen in Klammern bezeichnen die durchschnittliche Brennzeit in s.

Beispiele 4 bis 7

Ein Polycarbonat-Harz auf der Basis von Bisphenol A und Phosgen mit einer Schmelzflussrate von 7,5 g/10 min wurde mit wechselnden Mengen Natrium-tetrachloro-phthalimido-benzolsulfonat (TCPS) vermischt, stranggepresst, geformt und auf Schmelzfluss, kritische Dicke und Flammbeständigkeit geprüft. Tabelle II zeigt die Ergebnisse der Prüfung.

Tabelle II

| Beispiel | TCPS[a]-Salz Konzentration Gewichts-% | Schmelzfluss[b] g/ 10 min | Kritische Dicke mm(mils) | Flammbeständigkeit[c] (durchschnittl. Brennzeit in s) |
|---|---|---|---|---|
| Kontrolle | 0 | 7,7 | 4,83(190) | V-2 |
| 4 | 0,1 | 7,5 | 4,14(163) | V-O (2,9) |
| 5 | 0,17 | 7,5 | 3,89(153) | V-O (1,9) |
| 6 | 0,42 | 7,7 | —(——) | V-O (1,7) |
| 7 | 0,37 | 7,7 | 3,89(153) | V-O (1,6) |

[a] Natrium-tetrachloro-phthalimido-benzolsulfonat.

[b] gemessen nach ASTM D-1238.

[c] gemessen nach UL-94 an 3,2 mm (1/8″) dicken Proben.

Beispiel 8

Die thermische Stabilität des Natrium-tetrachloro-phthalimido-benzolsulfonat wurde thermogravimetrisch untersucht. Nach 30 min bei 300 °C wurde ein Gewichtsverlust von 10% festgestellt. N-Phenylphthalimid als Vergleichssubstanz verdampft nach 2,5 min bei 300 °C vollständig.

Somit verleihen gemäss der vorliegenden Erfindung die halogenierten aromatischen Imid-Sulfonate der Erfindung, wenn sie in Polycarbonate eingearbeitet werden, diesen flammenverzögernde Eigenschaften und erleiden nur einen unbedeutenden Gewichtsverlust beim Erhitzen auf normale Verarbeitungstemperaturen für Polycarbonate.

**Patentansprüche**

1. Verbindungen der allgemeinen Formel

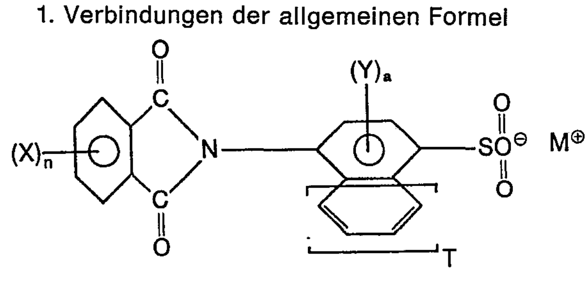

worin

X für ein Halogen,

n für 4,

Y für ein niederes Alkyl mit 1 bis 4 Kohlenstoff-Atomen oder ein Halogen-Atom,

a für eine ganze Zahl von 0 bis 4,

M für Natrium oder Kalium und

T für entweder 0 oder 1 stehen.

2. Eine Verbindung gemäss Anspruch 1, dadurch gekennzeichnet, dass sie folgenden Formeln entspricht

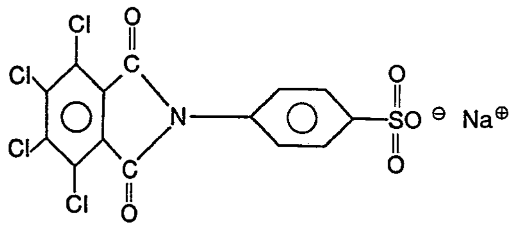

3. Verfahren zur Erhöhung der Flammbeständigkeit thermoplastischer aromatischer Polycarbonate, dadurch gekennzeichnet, dass den Polycarbonaten 0,01 bis 1 Gewichts-% einer Verbindung nach Anspruch 1 oder 2 zugemischt wird.

4. Verfahren gemäss Anspruch 3, dadurch gekennzeichnet, dass 0,1 bis 0,75 Gewichts-% einer Verbindung nach Anspruch 1 oder 2 zugemischt wird.

5. Thermoplastisches aromatisches Polycarbonat, dadurch gekennzeichnet, dass es ein Polycarbonat-Harz sowie 0,01 bis 1 Gewichts-% einer Verbindung nach Anspruch 1 oder 2 enthält.

6. Thermoplastisches aromatisches Polycarbonat, gemäss Anspruch 5, dadurch gekennzeichnet, dass es 0,1 bis 0,75 Gewichts-% einer Verbindung nach Anspruch 1 oder 2 enthält.

7. Thermoplastisches aromatisches Polycarbonat nach Anspruch 5 oder 6, dadurch gekennzeichnet, dass das genannte Polycarbonat-Harz sich von Bis-2-(4-hydroxyphenyl)-propan ableitet.

**Claims**

1. Compounds of the general formula

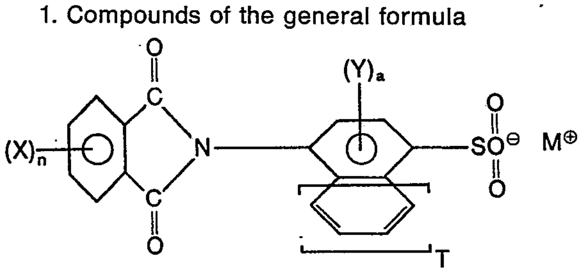

wherein

X represents a halogen,

n represents 4,

Y represents a lower alkyl having 1 to 4 carbon atoms or a halogen atom,

a represents an integer from 0 to 4,

M represents sodium or potassium and

T represents either 0 or 1.

2. A compound according to Claim 1, characterised in that it corresponds to the following formulae

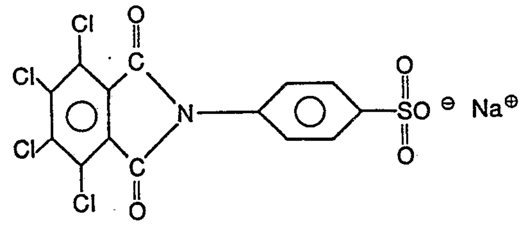

3. Method of increasing the flame resistance of thermoplastic aromatic polycarbonates, characterised in that 0.01 to 1% by weight of a compound according to Claim 1 or 2 is added to the polycarbonates.

4. Method according to Claim 3, characterised in that 0.1 to 0.75% by weight of a compound according to Claim 1 or 2 is added.

5. Thermoplastic aromatic polycarbonate, characterised in that it contains a polycarbonate resin and 0,01 to 1% by weight of a compound according to Claim 1 or 2.

6. Thermoplastic aromatic polycarbonate according to Claim 5, characterised in that it contains 0.1 to 0.75% by weight of a compound according to Claim 1 or 2.

7. Thermoplastic aromatic polycarbonate according to Claim 5 or 6, characterised in that the stated polycarbonate resin is derived from bis-2-(4-hydroxyphenyl)-propane.

**Revendications**

1. Composés de formule générale:

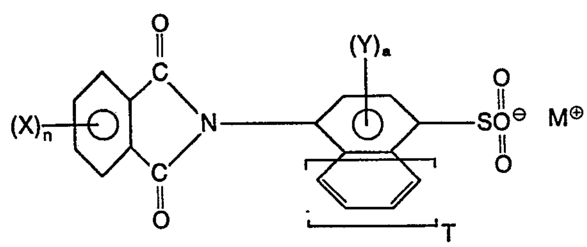

dans laquelle:

X représente un halogène,

n est égal à 4,

Y représente un groupe alkyle inférieur en $C_1$–$C_4$ ou un atome d'halogène,

a est un nombre entier de 0 à 4,

M représente le sodium ou le potassium, et

T est égal à 0 ou 1.

2. Un composé selon la revendication 1, caractérisé en ce qu'il répond à la formule suivante:

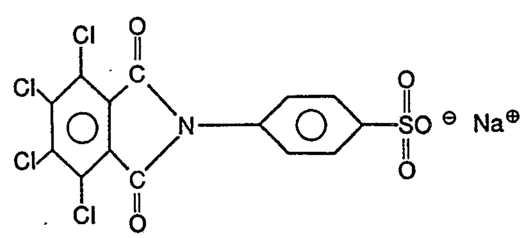

3. Procédé pour améliorer la résistance aux flammes de polycarbonates aromatiques thermoplastiques, caractérisé en ce que l'on mélange à ces polycarbonates de 0,01 à 1% en poids d'un composé selon la revendication 1 ou 2.

4. Procédé selon la revendication 3, caractérisé en ce que l'on mélange 0,1 à 0,75% en poids d'un composé selon la revendication 1 ou 2.

5. Polycarbonate aromatique thermoplastique, caractérisé en ce qu'il contient une résine de polycarbonate et 0,01 à 1% en poids d'un composé selon la revendication 1 ou 2.

6. Polycarbonate aromatique thermoplastique selon la revendication 5, caractérisé en ce qu'il contient de 0,1 à 0,75% en poids d'un composé selon la revendication 1 ou 2.

7. Polycarbonate aromatique thermoplastique selon la revendication 5 ou 6, caractérisé en ce que la résine de polycarbonate dérive du bis-2-(4-hydroxyphényl)-propane.